# EUROPEAN PATENT APPLICATION

(11) **EP 1 149 838 A1**
(43) Date of publication of application: **31.10.2001**
(21) Application number: 00201539.4
(22) Date of filing: 28.04.2000
(51) Int. Cl.: C07J 1/00

(54) **Estriol substantially free of 16alpha, 17alpha-estra-1,3,5(10)-triene-3,16,17-triol**

(71) Applicant: AZIENDE CHIMICHE RIUNITE ANGELINI FRANCESCO A.C.R.A.F. S.p.A., 00181 Roma (IT)
(72) Inventor: Giannangeli, Marilena, 00181 Roma (IT)
(74) Representative: Marchi, Massimo, Dr.

(57) **Abstract**

Estriol substantially free of 16α,17α-estra-1,3,5(10)-triene-3,16,17-triol, and process for obtaining the same.

## Description

The present invention relates to estriol substantially free of 16α,17α-estra-1,3,5(10)-triene-3,16,17-triol.

It is known that estriol, or 16α,17β-estra-1,3,5(10)-triene-3,16,17-triol, of formula (I) 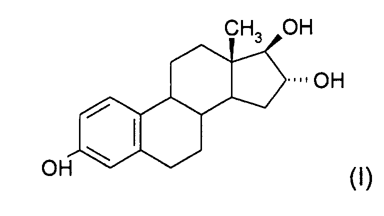 is prepared by reducing the estrone of formula (II) 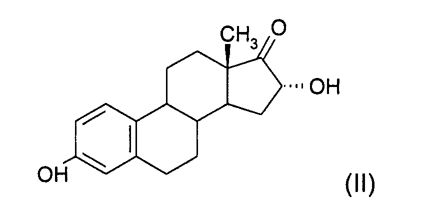 with sodium borohydride.

During this reduction, a certain amount of 16α,17α-estra-1,3,5(10)-triene-3,16,17-triol, of formula (III) 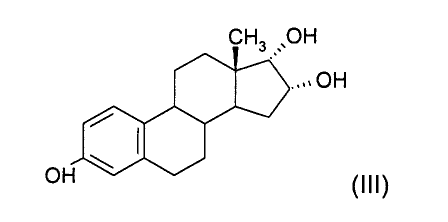 is also formed.

Very recently, however, it has been postulated that this compound of formula (III) might contribute to a possible carcinogenic action because of its vicinal diol nature without contributing at all to the therapeutic action since it is entirely inactive (Science, 279, 1631-2, 1998).

It has thus become necessary to find a source of estriol (I) free of the abovementioned compound of formula (III).

Now, it has been surprisingly found that this can be achieved by reducing the estrone of formula (II) with B-ipc-9-BBN or B-isopino-campheyl-9-borabicyclo[3.3. 1]nonane.

Therefore, it is a first object of the present invention to provide the estriol of formula (I) 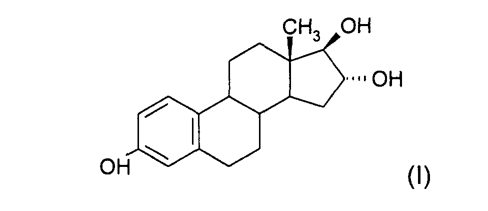 which is substantially free of 16α,17α-estra-1,3,5(10)-triene-3,16,17-triol of formula (III)

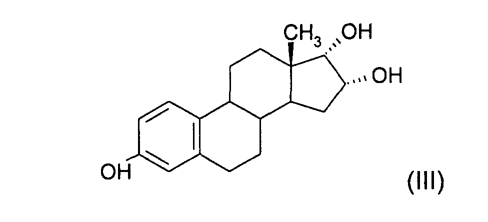

Further, it is a second object of the present invention to provide a method for reducing the estrone of formula (II) 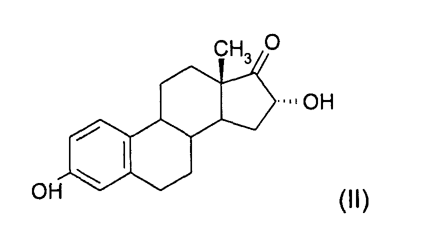 characterized in that the said reduction is carried out with B-ipc-9-BBN.

The example which follows is given to illustrate the present invention, without, however, limiting it in any way.

### EXAMPLE

B-ipc-9-BBN (12 mmol) and estrone (10 mmol) were loaded into a round-bottomed flask (50 ml) fitted with a magnetic stirrer, an addition funnel and a stream of nitrogen. The mixture was kept stirring at room temperature for 7 hours. After cooling the mixture to 0°C, acetaldehyde (3 mmol) was added and the mixture was left at room temperature for a further 1 hour. Next, ethyl ether (20 ml) and ethanolamine (12 mmol) were added and stirring was continued for a further 30 minutes. The boron complex precipitated was filtered off and the solution was concentrated.

Estriol was thus obtained free of 16α,17α-estra-1,3,5(10)-triene-3,16,17-triol, and had a melting point of 284-5°C.

The absence of 16α,17α-estra-1,3,5(10)-triene-3,16,17-triol was confirmed by HPLC analysis on a Supelcosil RP18 column, which showed an enantiomeric purity of 99% for the estriol obtained.

## Claims

1. Estriol of formula (I) 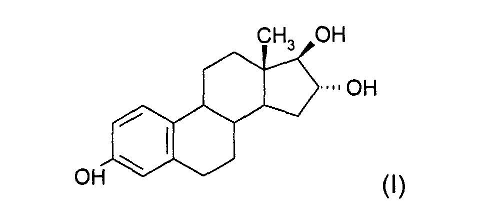 which is substantially free of 16α,17α-estra-1,3,5(10)-triene-3,16,17-triol of formula (III)

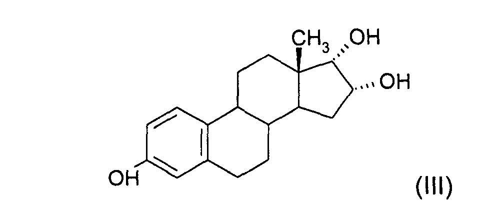

2. Method for reducing the estrone of formula (II) 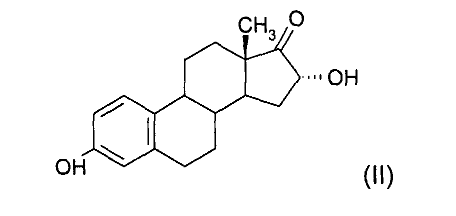 **characterized in that** the said reduction is carried out with B-ipc-9-BBN.
